(19) Europäisches Patentamt
European Patent Office
Office européen des brevets

(11) **EP 3 832 662 A1**

(12) **EUROPEAN PATENT APPLICATION**

(43) Date of publication:
**09.06.2021 Bulletin 2021/23**

(21) Application number: **19212842.9**

(22) Date of filing: **02.12.2019**

(51) Int Cl.:
*G16H 50/20* (2018.01)     *A61B 5/0285* (2006.01)
*A61B 5/055* (2006.01)     *A61B 5/107* (2006.01)
*A61B 6/00* (2006.01)      *A61B 8/08* (2006.01)
*A61B 5/20* (2006.01)      *A61B 18/00* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
**KH MA MD TN**

(71) Applicant: **Koninklijke Philips N.V.**
**5656 AG Eindhoven (NL)**

(72) Inventors:
• **FRACKOWIAK, Bruno Jean François**
**5656 AE Eindhoven (NL)**
• **MÜLLER, Manfred**
**5656 AE Eindhoven (NL)**
• **VAN DER HORST, Arjen**
**5656 AE Eindhoven (NL)**

(74) Representative: **Philips Intellectual Property &
Standards
High Tech Campus 5
5656 AE Eindhoven (NL)**

(54) **SYSTEMS AND METHODS FOR VASCULAR ASSESSMENT**

(57)     The invention provides an apparatus for determining a pulse wave velocity in a vessel of a subject. The apparatus includes a processor adapted to: obtain an initial pulse wave velocity measurement of a vessel; and obtain an image of the vessel. The image comprises a measurement location, corresponding to the location within the vessel at which the initial pulse wave velocity measurement is obtained, and a view of the vessel downstream of the measurement location.

The processor is further adapted to identify a geometric structure of the vessel and to derive physical parameters of the vessel based on the image of the vessel, which are used to generate a numerical model of the vessel characterizing the influence of the structure of the vessel and the physical parameters of the vessel on the initial pulse wave velocity measurement. The processor then adjusts the initial pulse wave velocity measurement based on the numerical model, thereby generating a pulse wave velocity output corrected upon reflection originating from downstream to the measurement location.

FIG. 5

EP 3 832 662 A1

## Description

FIELD OF THE INVENTION

The invention relates to the field of vascular assessment, and more specifically to the field of pulse wave velocity measurement.

BACKGROUND OF THE INVENTION

Pulse wave velocity measurements are a well-established way to measure the compliance of blood vessels. In particular, aortic and carotid pulse wave velocity is used to evaluate the risk of cardiovascular events.

The relationship between the compliance (C) of the vessel and the pulse wave velocity (PWV) can be described via the Moens-Korteweg equation:

$$PWV = \sqrt{V/C\rho},$$ wherein V is the vascular volume and $\rho$ is the blood density. This so called "reflection-free pulse wave velocity" depends only on the mechanical properties of the vessel and the fluid.

Typically, measuring the pulse wave velocity (PWV) includes determining the delay in arrival time between the pulses from one measurement location to the next. This method works best for long vessels such as the aorta as the pulse wave velocity is relatively high in arteries (i.e. 6 - 20 m/s) and therefore the distance should be large to accurately measure the time delay. The measured pulse wave velocity value is then a single value, spatial average pulse wave velocity, over a relatively long vessel length. PWV can be estimated using methods such as the sum of squares method and slope method according to the following equations:

$$PWV = \frac{1}{\rho}\sqrt{\frac{\sum dP^2}{\sum dU^2}}$$

Sum of square method

$$P = P_0 + \rho \times PWV \times U$$

Whater Hammer equation for the slope method, wherein: P is a pressure; and U is the wave speed.

Various examples of measuring PWV may be found, for example in: Boutouyrie, P. et al (2009), Assessment of pulse wave velocity, Artery Research, 3(1), pp.3-8; Khir, A.W et al (2001), Determination of wave speed and wave separation in the arteries, Journal of Biomechanics, 34 (2001), pp.1145-1155; and Davies, J. E. et al (2006), Use of simultaneous pressure and velocity measurements to estimate arterial wave speed at a single site in humans, AJP - Heart and Circulatory Physiology, 290, pp.878-885.

Pulse wave velocity measurements can vary along the length of a vessel due to the effect of wave reflections from bifurcations, stenoses or other structures with non-ideal impedance matching, even if the mechanical properties of the vessel are homogeneous. These reflected waves will influence the measured pulse wave velocity which will deviate from the reflection-free pulse wave velocity, defined above by Moens-Korteweg equation. In long, straight vessels such the aorta these deviations from the reflection-free pulse wave velocity are small, but if the measurement is done in close proximity to a strong reflection point, the measured pulse wave velocity can strongly deviate from the reflection-free pulse wave velocity. This is typically the case for short vessels like the renal arteries where a strong reflection point (the bifurcation at the end of the artery) is imminently close to the measurement point. In these vessels the measured pulse wave velocity cannot be used directly to assess the compliance of the vessel under measurement.

There is therefore a need for a means of accurately measuring the pulse wave velocity of a vessel regardless of the vessel structure.

SUMMARY OF THE INVENTION

The invention is defined by the claims.

According to examples in accordance with an aspect of the invention, there is provided an apparatus for determining a pulse wave velocity in a vessel of a subject, the apparatus comprising a processor adapted to:

obtain an initial pulse wave velocity measurement of a vessel;
obtain an image of the vessel, wherein the image represents both:

a measurement location within the vessel corresponding to the location at which the initial pulse wave velocity measurement is obtained; and

a view of the vessel downstream of the measurement location;

identify a geometric structure of the vessel based on the image of the vessel;
derive physical parameters of the vessel based on the image;
generate a numerical model of the vessel based on the structure of the vessel and the physical parameters of the vessel, wherein the numerical model characterizes an influence of the geometric structure of the vessel and the physical parameter of the vessel on the initial pulse wave velocity measurement; and
adjust the initial pulse wave velocity measurement based on the numerical model, thereby generating pulse wave velocity output corrected upon reflection originating from downstream to the measurement location.

The apparatus provides for a means of obtaining an adjusted pulse wave velocity measurement that accurately accounts for the geometric structure or further related physical properties of the vessel and the effects that these aspects can have on the initial measurement.

In particular, the apparatus provides for an accurate pulse wave velocity measurement in vessels where the geometric structure or related physical properties of the vessel changes in the vicinity of the measurement location.

In other words, the apparatus provides a means of accurately determining a pulse wave velocity in any vessel by automatically accounting for the geometric structure or further related physical properties of the vessel.

In an embodiment, the geometric structure of the vessel comprises a number of vessel branches in the downstream view. In particular the geometric structure of the vessel is a vessel network comprising a main branch, splitting into a first set of branches, which first set of braches split downstream into a second set of branches.

In this way, an accurate pulse wave velocity measurement may be obtained in vessels containing one or more branches downstream of the measurement location, which typically result in reflections (reflected waves) and decrease the accuracy of the pulse wave velocity measurement.

In preferred embodiments, the numerical model may account for a wide variety of physical properties of the vessel, the physical parameters of the vessel comprise two or more of the parameters selected from:

    a vessel length;
    a vessel diameter;
    a tapering of the vessel;
    a wall thickness of the vessel;
    a compliance of the vessel; and
    a resistance of the vessel;

wherein the length, diameter, tapering and wall thickness are parameters of the geometric structure (morphology), while compliance and resistance are further related physical parameters.

In an embodiment, the physical parameter comprises a vessel stiffness, which is in physical sense related to the compliance of the vessel, in particular the stiffer the vessel is, the less compliance it has.

The vessel stiffness is a key parameter of the pulse wave velocity measurement, meaning that accounting for it may result in a significant increase in the accuracy of the adjusted pulse wave velocity measurement.

In an embodiment, the processor is adapted to iteratively update the vessel stiffness based on the initial pulse wave velocity.

In this way, the derived vessel stiffness may be updated to better match the initial pulse wave velocity, thereby increasing the accuracy of the stiffness to the subject undergoing investigation and subsequent pulse wave velocity measurement adjustments.

In an embodiment, the numerical model comprises a modelled vascular resistance value, and wherein the processor is further adapted to:

    obtain pressure data relating to the blood pressure in the vessel and flow velocity data relating to the blood flow velocity in the vessel;
    generate an overall resistance value for the vessel based on the pressure data and the flow velocity data;
    compare the modelled vascular resistance value to the overall resistance value;
    generate a correction factor based on the comparison; and
    apply the correction factor to the numerical model.

In this way, the accuracy of the numerical model, and so the final adjusted pulse wave velocity measurement may be increased.

In an embodiment, the image of the vessel further comprises an upstream view, wherein the upstream view comprises a portion of the vessel upstream of the measurement location.

In this way, a larger range of vessel structures and physical properties may be accounted for in the numerical model, thereby increasing the accuracy of the adjusted pulse wave velocity measurement.

In an embodiment, the processor is further adapted to:

    evaluate an accuracy of the adjusted pulse wave velocity; and
    based on the accuracy, generate one or more of:

        a confidence value of the adjusted pulse wave velocity;
        an alert for informing a user of the accuracy;
        an instruction directing a user to obtain a further pulse wave velocity measurement; and
        a guidance instruction informing a user how to obtain a further pulse wave velocity measurement.

In this way, the apparatus may be adapted to self-evaluate the accuracy of the adjusted pulse wave velocity measurement in order to inform the user and/or advise the user as to how the measurement may be improved.

In an embodiment, the initial pulse wave velocity measurement is obtained from a renal vessel, and wherein processor is further adapted to classify the subject as responsive to renal denervation treatment if the adjusted pulse wave velocity is greater than or equal to a predetermined threshold.

In this way, the apparatus is adapted to stratify the eligibility of the subjects based on the likelihood that a subject will respond well to renal denervation treatment.

According to examples in accordance with an aspect of the invention, there is provided a system for determining a pulse wave velocity in a vessel, the system comprising:

an apparatus as described in any of the above embodiments;
a measurement device adapted to obtain the initial pulse wave velocity measurement from the vessel; and
an imaging device adapted to obtain the image of the vessel.

In an embodiment, the measurement device comprises one or more of:

a catheter;
a guidewire; and
an ultrasound device.

In an embodiment, the imaging device comprises one or more of:

an x-ray imaging device;
an ultrasound imaging device;
an MRI device;
an NMR imaging device; and
a PET imaging device.

In an embodiment, the system further comprises a display unit adapted to provide a visual representation of the adjusted pulse wave velocity measurement to the user.

According to examples in accordance with an aspect of the invention, there is provided a method for determining a pulse wave velocity in a vessel of a subject, the method comprising:

obtaining an initial pulse wave velocity measurement of a vessel;
obtaining an image of the vessel, wherein the image represents both:

a measurement location within the vessel corresponding to the location at which the initial pulse wave velocity measurement is obtained;
a view of the vessel downstream of the measurement location;

identifying a geometric structure of the vessel based on the image of the vessel;

derive physical parameters of the vessel based on the image;
generating a numerical model of the vessel based on the geometric structure of the vessel and the physical parameter of the vessel, wherein the numerical model characterizes an influence of the geometric structure of the vessel and the physical parameters of the vessel on the initial pulse wave velocity measurement; and
adjusting the initial pulse wave velocity measurement based on the numerical model, thereby generating pulse wave velocity output corrected upon reflection originating from downstream to the measurement location.

According to examples in accordance with an aspect of the invention, there is provided a computer program comprising computer program code means which is adapted, when said computer program is run on a computer, to implement the method described above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows a system for determining a pulse wave velocity in a vessel;
Figure 2 shows a flow diagram of the operations performed by the apparatus of Figure 1;
Figure 3 shows a graph of pulse wave velocity against distance from the measurement location;
Figure 4 shows a further example of the operations shown in Figure 2;
Figure 5 shows a schematic representation of the numerical model of the vessel;
Figure 6A shows a graph of vessel compliance against vessel radius;
Figure 6B shows a graph of vessel resistance against vessel radius;
Figure 7 shows a further example of the operations shown in Figure 2 and Figure 4.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Alterations and further modifications to the described devices, systems, and methods, and any further application of the principles of the present disclosure are fully contemplated and included within the present disclosure, in particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

The invention provides an apparatus for determining a pulse wave velocity in a vessel of a subject. The apparatus includes a processor adapted to: obtain an initial pulse wave velocity measurement of a vessel; and obtain an image of the vessel. The image represents a measurement location, corresponding to the location within the vessel at which the initial pulse wave velocity measurement was obtained, and a view of the vessel downstream of the measurement location.

The processor is further adapted to identify a geometric structure of the vessel and to derive physical parameters of the vessel based on the image of the vessel, which are used to generate a numerical model of the vessel characterizing the influence of the structure of the vessel and the physical parameter of the vessel on the initial pulse wave velocity measurement. The processor then adjusts the initial pulse wave velocity measurement based on the numerical model, thereby generating an adjusted pulse wave velocity.

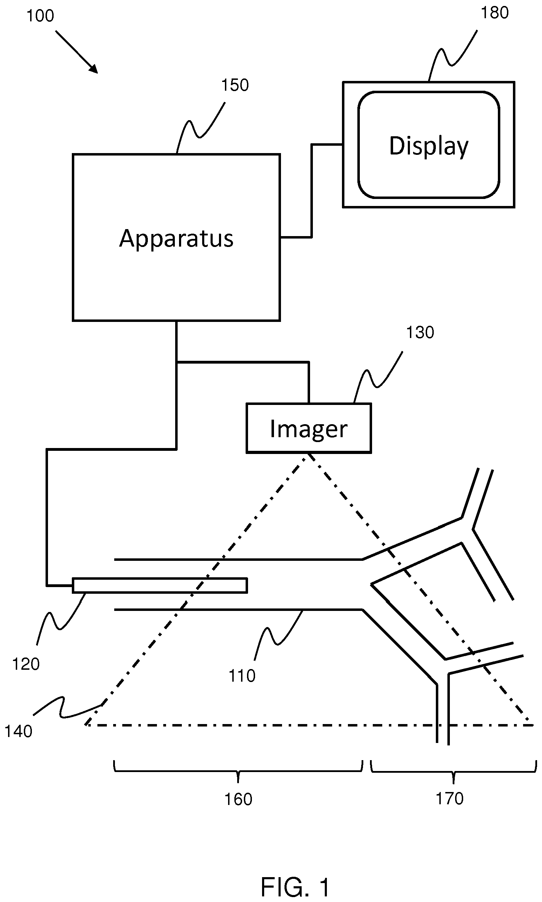

Figure 1 shows a system 100 for determining a pulse wave velocity in a vessel 110.

In the example shown in Figure 1, the system includes a measurement device 120 adapted to obtain an initial pulse wave velocity measurement from the vessel 110. The interventional device may be a catheter, a guidewire, a needle or any other measurement device that may be disposed within a vessel of a subject and obtain a pulse wave velocity measurement. In some embodiments the initial pulse wave velocity measurement is performed with one of the embodiments disclosed in the European patent application EP19212768 filed on Dec. 02, 2019, hereby incorporated by reference. Alternatively, or optionally an extracorporeal imaging device 130 may be used to measure pulse wave velocity of the vessel, e.g. as disclosed in WO2017198867 A1, hereby incorporated by reference.

Further, the system 100 in Figure 1 includes an imaging device 130 adapted to obtain an image of the vessel 110, the imaging device having a field of view 140 that includes a portion of the vessel. The imaging device may be any device capable of acquiring an image of a vessel of a subject, such as: an x-ray imaging device; an ultrasound imaging device; an MRI device; an NMR imaging device; and a PET imaging device. In some embodiments, the imaging device may be operated before any intervention by the measurement device to acquire anatomical information for generating an accurate 3D representation of the vessel tree.

The measurement device 120 may also include a marker, wherein the imaging device is adapted to recognize the marker as an indication of the measurement location of the initial pulse wave velocity measurement. For example, the marker may include a radiopaque marker for X-ray imaging device or other type of marker that can be visualized by respective imaging modality of the imaging device.

The initial pulse wave velocity measurement may be measured at a single point, or alternatively, the pulse wave velocity measurement may be measured over a plurality of measurement points, for example by way of a pull-back procedure using a catheter and guidewire. Using a plurality of measurement points may increase the accuracy of the initial pulse wave velocity and so increase the accuracy of the final adjusted pulse wave velocity measurement. Further, the measurement point may be a volume and the initial pulse wave velocity measurement need not be constant over the measurement volume.

It should be noted that the imaging device 130 is not required to acquire the image of the vessel 110 simultaneously with the initial pulse wave velocity measurement. For example, the imaging device 130 may take an image of the vessel 110 at one point in time and may determine the measurement location of the initial pulse wave velocity measurement at another point in time. In this way, suitable settings and methods, such as contrast enhancing methods for the vessel tree, for both types of imaging may be employed.

The system 100 includes an apparatus 150 for determining a pulse wave velocity in the vessel 110 of a subject. The apparatus comprises a processor adapted to perform the operations described herein. The processor may be any suitable processor.

The apparatus 150 is adapted to obtain an initial pulse wave velocity measurement of the vessel 110 and an image of the vessel. The apparatus 150 may obtain the initial pulse wave velocity measurement and the image of the vessel directly from the measurement device and the imaging device, respectively. Alternatively, the apparatus may obtain the initial pulse wave velocity measurement and the image of the vessel from a memory of the system.

The image of the vessel represents both a measurement location 160 within the vessel 110, corresponding to the location at which the initial pulse wave velocity measurement is obtained, and a downstream view 170 of the vessel. In addition, the image of the vessel may further comprise an upstream view, comprising a view of the vessel upstream of the measurement location.

The apparatus 150 is adapted to identify a geometric structure of the vessel based on the image of the vessel. The apparatus may employ any suitable image processing technique in order to identify the geometric structure of the vessel. For example, segmentation and/or shape recognition algorithms may be applied to the image in order to derive the geometric structure of the vessel. The geometric structure of the vessel may relate to the number of vessel branches in the downstream view. In other words, the branches of the vessel may be derived from the image of the vessel. Further, the geometric structure may include stenoses or any other structure with poor impendence matching.

The apparatus 150 is further adapted to identify a physical parameter of the vessel based on the image. As above, any suitable image processing means may be applied to the image in order to derive a physical parameter of the vessel. The physical parameter of the vessel may comprise any one or more of: a vessel length; a vessel diameter; a tapering of the vessel; a wall thickness of the vessel; a compliance (or stiffness) of the vessel; and a resistance of the vessel.

Based on the identified geometric structure of the vessel and the identified physical parameter of the vessel, the apparatus 150 is adapted to generate a numerical model of the vessel.

The numerical model corresponds to an influence of the geometric structure of the vessel and the physical parameter of the vessel on the initial pulse wave velocity measurement. In other words, the numerical model simulates the effects that the geometric structure and physical properties of the vessel would have on a pulse wave velocity measurement, thereby allowing for such effects to be accounted for when adjusting the initial pulse wave velocity measurement in order to arrive the adjusted pulse wave velocity measurement. Thus, the adjusted pulse wave velocity measurement represents the reflection-free pulse wave velocity of the vessel.

Put another way, the apparatus 150 generates a numerical model of the vascular tree (the geometric structure of the vessel) from the image of the vessel, which calculates the expected reflections of pressure and flow waves in the vessel and how they influence the initial pulse wave velocity measurement.

The apparatus 150 is adapted to adjust the initial pulse wave velocity measurement based on the numerical model, thereby generating the adjusted pulse wave velocity. In other words, based on the numerical model, the apparatus determines a correction that correlates the measured initial pulse wave velocity measurement with the true, adjusted pulse wave velocity measurement.

The system 100 of Figure 1 further includes a display unit adapted to provide a visual representation of the adjusted pulse wave velocity measurement to the user.

For example, the display unit may display a single adjusted pulse wave velocity measurement value to the user. Alternatively, or in addition, the apparatus 150 may be adapted to generate a map of adjusted pulse wave velocity measurements for the vessel, and further, for one or more of the vasculature branches within the downstream portion 170. Such a map of adjusted pulse wave velocity measurements may be overlaid on the image of the vessel for displaying to the user in the appropriate anatomical context.

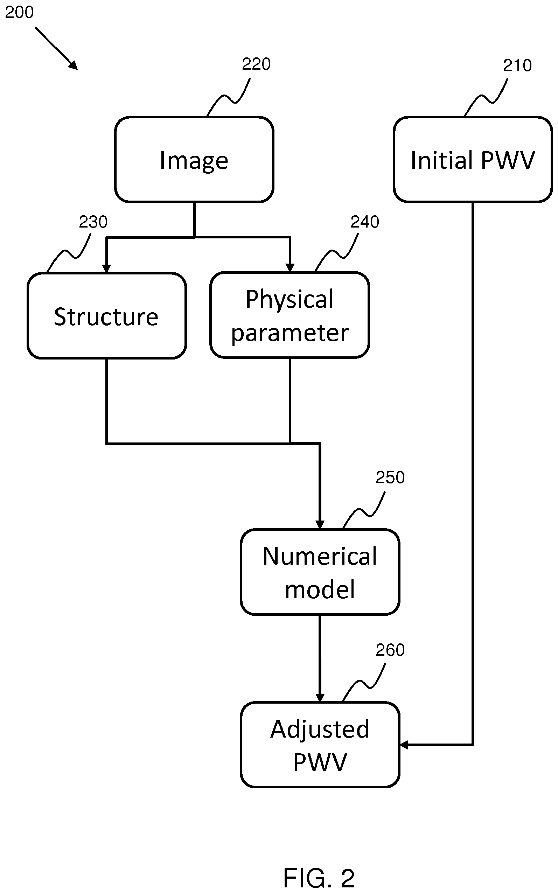

Figure 2 shows a flow diagram 200 of the operations performed by the apparatus 150 of Figure 1.

In step 210, an initial pulse wave velocity measurement of a vessel is obtained and in step 220, an image of the vessel is obtained, wherein the image represents a measurement location and a downstream view as described above. The pulse wave velocity measurement and obtaining an image of the vessel is already described with regard to the system.

In step 230, a geometric structure of the vessel is identified based on the image of the vessel and in step 240 physical parameters of the vessel are derived based on the image.

In step 250, a numerical model of the vessel is generated based on the geometric structure of the vessel and the physical parameters of the vessel, wherein the numerical model characterizes an influence of the structure of the vessel and the physical parameters of the vessel on the initial pulse wave velocity measurement.

In step 260, the initial pulse wave velocity measurement is adjusted based on the numerical model, thereby generating the pulse wave velocity output corrected upon reflection downstream to the measurement location.

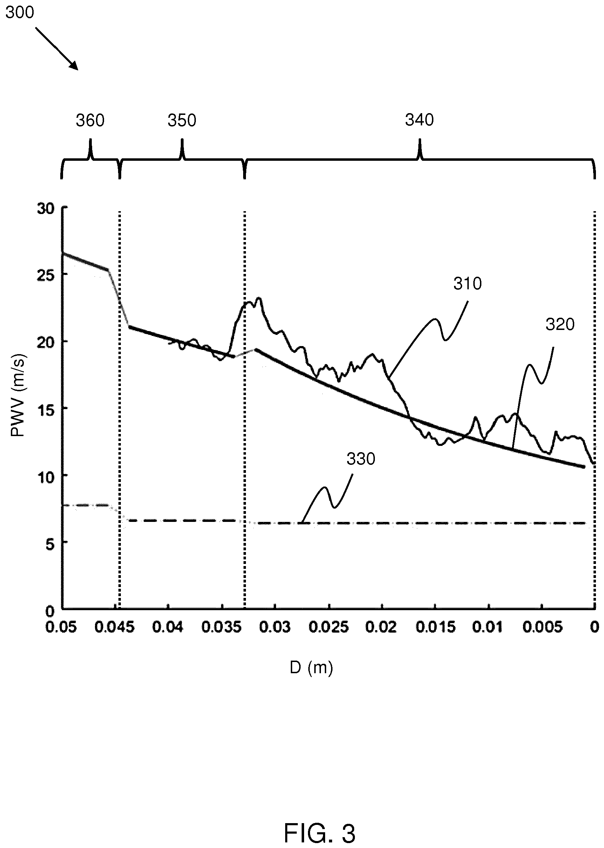

Figure 3 shows a graph 300 of pulse wave velocity (PWV) against distance (D) from the measurement location comparing a plot 310 of initial pulse wave velocity measurements to a plot 320 of the corresponding adjusted pulse wave velocity measurements following the operations of Figure 2. Figure 3 further shows a plot 330 of the pulse wave velocity output corrected upon reflection downstream to the measurement location, which can be regarded as reflection-free PWV, which is calculated from the numerical model, for example, by way of the Moens-Korteweg equation relating to the vessel stiffness.

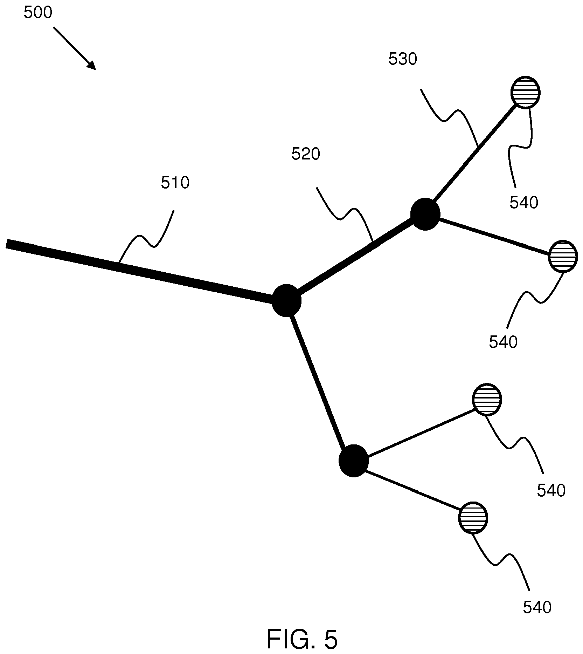

As can be seen from Figure 3, the plot 320 of the adjusted pulse wave velocity measurements is separated into three sections 340, 350 and 360, which represent different branches of the vessel under investigation. More specifically, section 340 represents the main branch of the vessel wherein the initial pulse wave velocity measurement is taken. Section 350 represents a primary branch coming off the main branch of the vessel and section 360 represents a secondary branch coming off the primary branch. Figure 5 gives a schematic illustration further elucidating the terminology used with respect to the downstream vessel network.

In the examples described above, one of the physical parameters of the vessel that may be used in the generation of the numerical model is a vessel stiffness. As the physical parameters are estimated from the image of the vessel, the vessel stiffness may also be estimated from the image.

In other words, the vasculature model may be built using angiography image input to determine the vessel dimensions, such as: wall thickness (h); vessel radius (r); and vessel length (L). The wall stiffness of the vessel (E) may be estimated from the generic correlation described in Olufsen et al "Structured tree outflow condition for blood flow in larger systemic arteries", The American Physiological Society, 1999, wherein:

$$\frac{Eh}{r} = k_1 e^{(k_2 r)} + k_3 \,,$$

wherein: $k_1 = 2e7 \, Nm^{-2}$; $k_2 = -2.253 \, mm^{-1}$; and $k_3 = 8.65e5 \, Nm^{-2}$.

The correlation given above does not take into account physiological variation, especially arterial stiffening and vasoconstriction due to overdrive in the sympathetic nerve. Moreover, the correlation has been determined from averaged population data, meaning that the individuality of each subject may result in some variability in the vessel stiffness parameter.

It can be observed from Figure 3, that the numerical model is capable of providing reflection-free PWV along the entire length of the geometrical structure of the vessel that was obtained by the imaging device 130, and which geometrical structure was used in the numerical model. Furthermore, the numerical model is suitable to provide discrete outputs at predetermined locations along the geometrical structure of the vessel, based on a measured PWV at an initial location in the vessel, the geometrical structure of the vessel and the derived physical parameters.

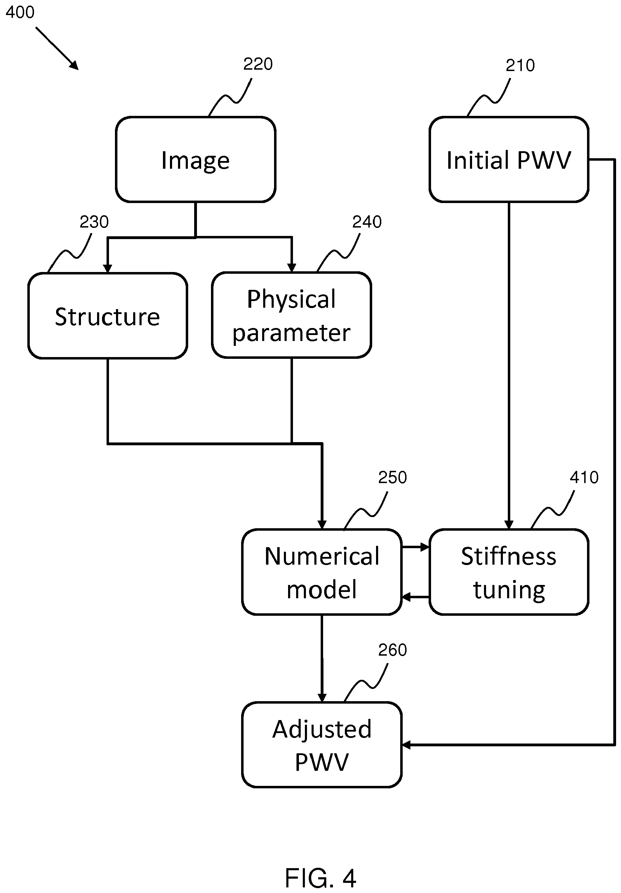

Figure 4 shows a further example 400 of the operations shown in Figure 2.

In addition to those operations described above with reference to Figure 2, the apparatus is further adapted to iteratively tune the simulated vessel stiffness of the numerical model based on the initial pulse wave velocity measurement, which may be used to optimize the *Eh* product value in cases where the value of *h* cannot be accurately determined directly.

In step 410, a simulated vessel stiffness is updated based on the initial pulse wave velocity.

Put another way, the apparatus is adapted to performed an iterative process step to match the simulated pulse wave velocity profile of the numerical model to the measured pulse wave velocity by tuning the simulated vessel stiffness, for example of the simulated main branch of the vessel. This provides more accurate results in terms of the adjusted pulse wave velocity and further provides an estimation of the arterial stiffening effect when compared with the stiffness value of the vessel under different medicament induced regimes or stimulations.

In the examples described above, the numerical model is constructed using angiography image input to determine the physical parameters of the vessel, such as the vessel dimensions: wall thickness h; the vessel diameter d; and vessel length L.

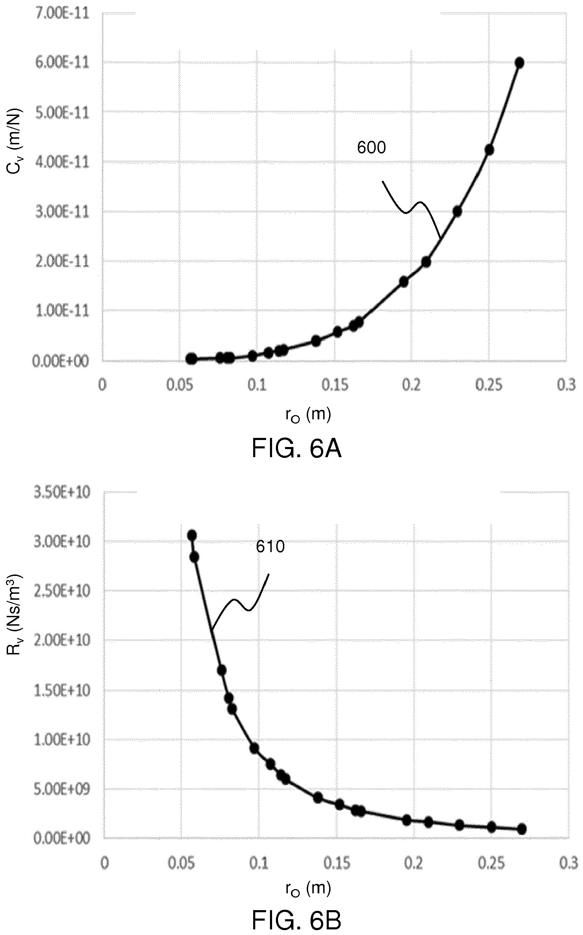

Figure 5 shows a schematic representation 500 of vessel network used exemplarily for the numerical model. In particular the geometric structure of the vessel can be regarded as a vessel network comprising a main branch, splitting into a first set of branches, which first set of braches split further downstream into a second set of branches. Accordingly, the numerical model comprises a main branch 510, a first primary branch 520 and a second primary branch 530. There is an outlet boundary 540, which may have an outlet boundary condition equivalent to a Windkessel 0D model, wherein the impedance of the outlet boundary is dependent on the resistance ($R_v$) and compliance ($C_v$) of the vessel, which in turn are outlet radius (ro) dependent as shown by the plot 600 of Figure 6A and the plot of 610 of Figure 6B, respectively. The relationships of the model may be determined from a Structured Fractal Tree Model (SFTM) as described in Olufsen et al "Structured tree outflow condition for blood flow in larger systemic arteries", The American Physiological Society, 1999.

The SFTM is generated for resting conditions and does not take into account the vasoconstriction due to the overdrive in the sympathetic nerve. The compliance doesn't significantly influence the pulse wave velocity in the main branch of the vessel; however, even a slight change in the downstream vasculature radius can have a significant effect on the overall resistance, which influences the pulse wave velocity profile in the main branch.

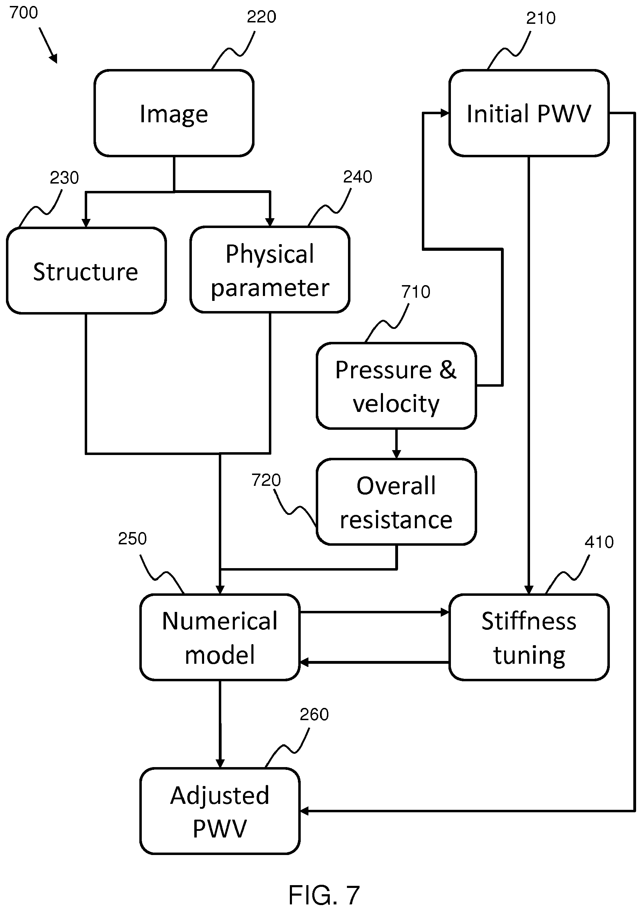

Figure 7 shows a further example 700 of the operations shown in Figure 2 and Figure 4.

In addition to those operations described above with reference to Figure 2 and Figure 4, the apparatus is further adapted to obtain 710 pressure data relating to the blood pressure in the vessel and flow velocity data relating to the blood flow velocity in the vessel and generate 720 an overall resistance value for the vessel based on the pressure data and the flow velocity data.

In other words, the value of the overall vasculature resistance may be estimated from blood flow and velocity measurements, which may also be used for the initial pulse wave velocity measurement in the main branch of the vessel.

This overall resistance value is compared to the one derived from the SFTM model described above, which may then be used to generate a correction factor to be applied to each outlet of the vasculature model.

Put another way, a correction factor for the numerical model may be generated by comparing the measured overall vascular resistance to the vascular resistance modelled in the numerical model. The correction factor may improve the accuracy of the numerical model to a given subject, thereby increasing the accuracy of the adjusted pulse wave velocity measurement.

The pressure and velocity are measured in the vessel branch to estimate the overall vasculature resistance. Further, the time varying pressure and velocity profiles may be measured. Accordingly, instead of a single point pulse wave velocity value, the time-varying pressure and velocity values over the pulse may be compared to the numerical model outcome in order to generate the correction factor, thereby increasing the accuracy of the adjusted pulse wave velocity measurement.

It should be noted that the operations introduced in Figure 7 may be performed without the additional operations of Figure 4. In other words, the apparatus may be adapted to perform the operations of Figure 2 with the additional steps of 710 and 720 only.

As discussed above, the numerical model is constructed using image derived data, such as: vessel length; vessel diameter; vessel wall thickness; outlet boundary resistance; and outlet boundary compliance values, as an input. Those input data have a certain variability, which generates some spread in the simulation outcome. For example, vessel length and diameter are directly extracted from the angiography image, which can result in a significant spread in the simulation outcome.

Nevertheless, the iterative tuning process of the vessel stiffness described above with reference to Figure 4 may compensate for this variability, thereby bringing the simulated pulse wave velocity profile close to the experimentally obtained one.

Accordingly, the apparatus may be further adapted to evaluate the accuracy of the adjusted pulse wave velocity based on the operations performed. Put another way, a sensitivity analysis is implemented in the apparatus and a confidence interval may be derived next to the adjusted pulse wave velocity measurement. The confidence interval may be any representation suitable for indicating an accuracy of the adjusted pulse wave velocity to the user, such as: a numerical indication; a color-based indication; a visual indication; an audible indication; and the like. Further, the system may be adapted to generate an alert based on the accuracy assessment, for example when the accuracy falls below a predetermined threshold.

Further optionally, the system may recognize when measurements are inaccurate due to suboptimal data acquisition locations or when the measurement falls into a pre-defined gray zone. For example, if the measurement is performed at a location where the pulse wave velocity varies significantly over the length of the vessel (typically close to a bifurcation), the system may consider the location as suboptimal and prompt the user for further measurements/actions to enhance accuracy of the adjusted pulse wave velocity measurement. For example, the system may generate an instruction directing the user to obtain a further pulse wave velocity measurement and/or a guidance instruction informing a user how to obtain the further pulse wave velocity measurement. Alternatively, it is contemplated that based on the confidence interval no alert is prompted, but the system directly generates an instruction to the user to obtain a further pulse wave velocity measurement and/or a guidance instruction informing a user how to obtain the further pulse wave velocity measurement. In some embodiments the guidance information is based on mismatch of impedance before and after bifurcation. Impedance of the vessel is defined as

$$Z \approx \sqrt{\frac{L_{ind}}{C}} = 1/A \sqrt{\frac{\rho E h}{d}},$$

where $L_{ind}$ is the inductance $L_{ind} = \frac{L \times \rho}{A}$ and C is the compliance $C = \frac{L \times A \times d}{E \times h}$. If the impedance of the distal portion of the vessel is equal to that of the proximal portion, then the impedance is matched. The concept with RLC boundary indicates that:

- with +50 % mismatch, there is approximately 0.5 m/s overestimation at the branch;
- with +100 % mismatch, there is approximately 2.3 m/s overestimation at the branch.

If the PWV value is close to the threshold value used for stratification of subjects according to eligibility for a treatment (e.g. for renal denervation treatment), then in optimal situation the user is prompted to move further away from the bifurcation.

The systems and methods above may be employed for performing pulse wave velocity measurements in renal vessels, which are typically difficult to obtain accurate pulse wave velocity measurements in due to the high number of vessel branches.

Pulse wave velocity measurements in the renal arteries can help in subject stratification for renal denervation, i.e. determining whether a subject would benefit from renal denervation. This is because pulse wave velocity measurements are influenced by the activity of the sympathetic nerves around the renal artery. A high pulse wave velocity may be an indication of sympathetic overdrive and patients with a sympathetic overdrive are likely to benefit from renal denervation.

Quantitatively, pulse wave velocity measurements larger than approximately 10 m/s may be taken as an indication that a patient is likely to respond to renal denervation therapy. Thus, the apparatus may be adapted to apply a predetermined threshold, for example of 10 m/s, to compare the adjusted pulse wave velocity measurements in order to automatically identify suitable subjects for renal denervation.

Further, a stimulus may also be used to quantify the effect of the renal sympathetic overdrive, for example by providing a stimulus such as an alpha blocker.

The predetermined threshold value for determining whether a subject would be responsive to renal denervation may then be determined based on the difference, or ratio, between the adjusted PWV before and after the stimulus is provided. The stimulus may include one or more of: a physical manoeuver; a pharmaceutical stimulus; and an electrical stimulus.

In particular, such stimuli may include: a vasodilating drug, such as dopamine, nitrate, adenosine and the like; a nerve neuromodulating drug, such as an anesthetic, a temporary sympathetic nerve blocking drug, or a sympathetic nerve stimulating drug; or a handgrip administered before or during the measurement; or any other stimulus affecting sympathetic nerve response.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

**Claims**

1. An apparatus (150) for determining a pulse wave velocity in a vessel (110) of a subject, the apparatus comprising a processor adapted to:

   obtain an initial pulse wave velocity measurement of a vessel;
   obtain an image of the vessel, wherein the image represents both:

   a measurement location (160) within the vessel corresponding to the location at which the initial pulse wave velocity measurement is obtained; and
   a view (170) of the vessel downstream of the measurement location;

   identify a geometric structure of the vessel based on the image of the vessel;

   derive physical parameters of the vessel based on the image;
   generate a numerical model of the vessel based on the geometric structure of the vessel and the physical parameters of the vessel, wherein the numerical model characterizes an influence of the geometric structure of the vessel and the physical parameters of the vessel on the initial pulse wave velocity measurement; and
   adjust the initial pulse wave velocity measurement based on the numerical model, thereby generating pulse wave velocity output corrected upon reflection originating from downstream to the measurement location.

2. The apparatus (150) as claimed in claim 1, wherein the geometric structure of the vessel comprises a plurality of vessel branches in the downstream view.

3. The apparatus (150) as claimed in claim 1 or 2, wherein the physical parameters of the vessel are selected from:

   a vessel length;
   a vessel diameter;
   a tapering of the vessel;
   a wall thickness of the vessel;
   a compliance of the vessel; and
   a resistance of the vessel.

4. The apparatus (150) as claimed in any of claims 1 to 3, wherein the physical parameters comprise a vessel stiffness.

5. The apparatus (150) as claimed in claim 4, wherein the processor is adapted to iteratively update the vessel stiffness based on the initial pulse wave velocity.

6. The apparatus (150) as claimed in any of claims 1 to 5, wherein the numerical model comprises a modelled vascular resistance value, and wherein the processor is further adapted to:

   obtain pressure data relating to the blood pressure in the vessel and flow velocity data relating to the blood flow velocity in the vessel;
   generate an overall resistance value for the vessel based on the pressure data and the flow velocity data;
   compare the modelled vascular resistance value to the overall resistance value;
   generate a correction factor based on the comparison; and
   apply the correction factor to the numerical model.

7. The apparatus (150) as claimed in any of claims 1 to 6, wherein the image of the vessel further comprises a view of the vessel upstream of the measurement location.

8. The apparatus (150) as claimed in any of claims 1 to 7, wherein the processor is further adapted to:

evaluate an accuracy of the adjusted pulse wave velocity; and
based on the accuracy, generate one or more of:

a confidence value of the adjusted pulse wave velocity;
an alert for informing a user of the accuracy;
an instruction directing a user to obtain a further pulse wave velocity measurement; and
a guidance instruction informing a user how to obtain a further pulse wave velocity measurement.

9. The apparatus (150) as claimed in any of claims 1 to 8, wherein the initial pulse wave velocity measurement is obtained from a renal vessel, and wherein processor is further adapted to classify the subject as eligible to renal denervation treatment if the adjusted pulse wave velocity is greater than or equal to a predetermined threshold.

10. A system (100) for determining a pulse wave velocity in a vessel, the system comprising:

an apparatus (150) as claimed in claim 1 to 9;
at least a device configured to provide:

the initial pulse wave velocity measurement from the vessel; and
an image of the vessel.

11. The system (100) as claimed in claim 10, wherein the device comprises one of:
an intravascular device (120) and an extracorporeal imaging device (130) configured for providing the initial pulse wave velocity measurement from the vessel.

12. The system (100) as claimed in any of claims 10 to 11, wherein the device comprises one or more of:

an x-ray imaging device;
an ultrasound imaging device;
an MRI device;
an NMR imaging device; and
a PET imaging device;

configured for providing the image of the vessel.

13. The system (100) as claimed in any of claims 10 to 12, wherein the system further comprises a display unit adapted to provide a visual representation of the adjusted pulse wave velocity output to the user.

14. A method (200) of determining a pulse wave velocity in a vessel of a subject, the method comprising:

obtaining (210) an initial pulse wave velocity measurement of a vessel;
obtaining (220) an image of the vessel, wherein the image represents both:

a measurement location within the vessel corresponding to the location at which the initial pulse wave velocity measurement is obtained; and
a view of the vessel downstream of the measurement location;

identifying (230) a geometric structure of the vessel based on the image of the vessel;
deriving (240) physical parameters of the vessel based on the image;
generating (250) a numerical model of the vessel based on the geometric structure of the vessel and the physical parameter of the vessel, wherein the numerical model characterizes an influence of the geometric structure of the vessel and the physical parameter of the vessel on the initial pulse wave velocity measurement; and
adjusting (260) the initial pulse wave velocity measurement based on the numerical model, thereby generating pulse wave velocity output corrected upon reflection originating from downstream to the measurement location.

15. A computer program comprising computer program code means which is adapted, when said computer program is run on a computer, to implement the method of claim 14.

100

180

150

Display

Apparatus

130

Imager

120

110

140

160

170

FIG. 1

FIG. 2

EP 3 832 662 A1

FIG. 3

13

400

220
Image

210
Initial PWV

230
Structure

240
Physical parameter

250
Numerical model

410
Stiffness tuning

260
Adjusted PWV

FIG. 4

FIG. 5

FIG. 6A

FIG. 6B

700

FIG. 7

Europäisches
Patentamt

European
Patent Office

Office européen
des brevets

## EUROPEAN SEARCH REPORT

Application Number

EP 19 21 2842

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PATRICK SEGERS ET AL: "Wave reflection leads to over- and underestimation of local wave speed by the PU- and QA-loop methods: theoretical basis and solution to the problem", PHYSIOLOGICAL MEASUREMENT, INSTITUTE OF PHYSICS PUBLISHING, BRISTOL, GB, vol. 35, no. 5, 8 April 2014 (2014-04-08), pages 847-861, XP020262373, ISSN: 0967-3334, DOI: 10.1088/0967-3334/35/5/847 [retrieved on 2014-04-08] | 1-8, 10-15 | INV. G16H50/20 A61B5/0285 A61B5/055 A61B5/107 A61B6/00 A61B8/08 A61B5/20  ADD. A61B18/00 |
| A | * case (ii) and section "Data Processing" on page 855; figure 5; table 1 * | 9 | |
| A | ABIGAIL SWILLENS ET AL: "Comparison of Non-Invasive Methods for Measurement of Local Pulse Wave Velocity Using FSI-Simulations and In Vivo Data", ANNALS OF BIOMEDICAL ENGINEERING, vol. 41, no. 7, 1 July 2013 (2013-07-01), pages 1567-1578, XP55698105, New York ISSN: 0090-6964, DOI: 10.1007/s10439-012-0688-z * section "Carotid Artery Geometry"; figure 1(b) * | 1-15 | TECHNICAL FIELDS SEARCHED (IPC)  G16H A61B |
| A | US 2019/290139 A1 (SIO CHARLES FREDERIK [NL] ET AL) 26 September 2019 (2019-09-26) * paragraph [0071]; figure 7 * | 1-15 | |
| A | US 2005/154299 A1 (HOCTOR RALPH T [US] ET AL) 14 July 2005 (2005-07-14) * paragraphs [0083], [0084] * | 1-15 | |

-/--

The present search report has been drawn up for all claims

| Place of search | Date of completion of the search | Examiner |
|---|---|---|
| The Hague | 28 May 2020 | Mecking, Nikolai |

EPO FORM 1503 03.82 (P04C01)

page 1 of 2

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SWILLENS A ET AL: "Accuracy of Carotid Strain Estimates From Ultrasonic Wall Tracking: A Study Based on Multiphysics Simulations and In Vivo Data", IEEE TRANSACTIONS ON MEDICAL IMAGING, IEEE SERVICE CENTER, PISCATAWAY, NJ, US, vol. 31, no. 1, 30 December 2011 (2011-12-30), pages 131-139, XP011491010, ISSN: 0278-0062, DOI: 10.1109/TMI.2011.2165959 * section II.A.; figure 1 * | 1-15 | |

TECHNICAL FIELDS
SEARCHED      (IPC)

The present search report has been drawn up for all claims

| Place of search | Date of completion of the search | Examiner |
|---|---|---|
| The Hague | 28 May 2020 | Mecking, Nikolai |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 21 2842

This annex lists the patent family members relating to the patent documents cited in the above-mentioned European search report.
The members are as contained in the European Patent Office EDP file on
The European Patent Office is in no way liable for these particulars which are merely given for the purpose of information.

28-05-2020

| Patent document cited in search report | | Publication date | Patent family member(s) | | Publication date |
|---|---|---|---|---|---|
| US 2019290139 | A1 | 26-09-2019 | CN | 109152540 A | 04-01-2019 |
| | | | EP | 3457927 A1 | 27-03-2019 |
| | | | JP | 2019518519 A | 04-07-2019 |
| | | | US | 2019290139 A1 | 26-09-2019 |
| | | | WO | 2017198813 A1 | 23-11-2017 |
| US 2005154299 | A1 | 14-07-2005 | NONE | | |

For more details about this annex : see Official Journal of the European Patent Office, No. 12/82

EPO FORM P0459

## REFERENCES CITED IN THE DESCRIPTION

### Patent documents cited in the description

- EP 19212768 A

- WO 2017198867 A1

### Non-patent literature cited in the description

- **BOUTOUYRIE, P. et al.** Assessment of pulse wave velocity. *Artery Research,* 2009, vol. 3 (1), 3-8
- **KHIR, A.W et al.** Determination of wave speed and wave separation in the arteries. *Journal of Biomechanics,* 2001, vol. 34, 1145-1155

- **DAVIES, J. E. et al.** Use of simultaneous pressure and velocity measurements to estimate arterial wave speed at a single site in humans. *AJP - Heart and Circulatory Physiology,* 2006, vol. 290, 878-885
- **OLUFSEN et al.** Structured tree outflow condition for blood flow in larger systemic arteries. *The American Physiological Society,* 1999